(19) Europäisches Patentamt

European Patent Office

Office européen des brevets

(11) **EP 3 607 997 A1**

(12) **DEMANDE DE BREVET EUROPEEN**

(43) Date de publication:
**12.02.2020 Bulletin 2020/07**

(51) Int Cl.:
*A61Q 19/08* (2006.01)    *A61K 8/9794* (2017.01)

(21) Numéro de dépôt: **19190801.1**

(22) Date de dépôt: **08.08.2019**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(30) Priorité: **10.08.2018   LU 100904**

(71) Demandeur: **Société De Recherche Cosmétique
S.à.r.L.
2314 Luxembourg (LU)**

(72) Inventeurs:
• **MERINVILLE, Eve
L-1618 LUXEMBOURG (LU)**
• **ELKAIM, Judith
L-2561 LUXEMBOURG (LU)**

(74) Mandataire: **Petit, Maxime et al
Ipsilon
Le Centralis
63, avenue du Général Leclerc
92340 Bourg-la-Reine (FR)**

(54) **COMPOSITION COSMETIQUE COMPRENANT UN EXTRAIT DE PASSIFLORE ET DES CELLULES D'EDELWEISS ET UTILISATIONS**

(57)     La présente invention est relative à une nouvelle composition cosmétique ou dermatologique, à usage topique ou oral, comprenant au moins un extrait de passiflore, au moins des cellules de callus d'edelweiss, et au moins un support physiologiquement acceptable, à un procédé de traitement cosmétique non thérapeutique de la peau mettant en oeuvre une telle composition cosmétique, ainsi qu'à l'utilisation non thérapeutique d'une telle composition cosmétique pour protéger la peau des agressions de l'environnement, prévenir, retarder et/ou combattre les signes du vieillissement cutané.

EP 3 607 997 A1

**Description**

La présente invention se rapporte au domaine technique général des produits cosmétiques ou dermatologiques, à usage topique ou oral, utilisables notamment sur la peau.

Plus précisément, la présente invention est relative à une nouvelle composition cosmétique à application topique comprenant au moins un extrait de passiflore, des cellules de culture de callus d'edelweiss et/ou un extrait de cellules de culture de callus d'edelweiss et au moins un support physiologiquement acceptable, à un procédé de traitement cosmétique non thérapeutique de la peau mettant en oeuvre une telle composition cosmétique, ainsi qu'à l'utilisation non thérapeutique d'une telle composition cosmétique pour prévenir et/ou retarder les signes du vieillissement cutané.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et, par conséquent, il joue un rôle fondamental pour assurer la protection et le maintien d'une bonne trophicité. C'est pourquoi de nombreuses compositions ont été mises au point afin de le protéger, notamment des agressions de l'environnement.

Le derme est la couche la plus épaisse, riche en nerfs, en vaisseaux sanguins et en glandes sudoripares, et se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules, comme les macrophages et les leucocytes, sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque ou extrinsèque, c'est-à-dire provoqué par l'environnement, y compris les agressions climatiques, qui peuvent notamment contribuer à accélérer la dégradation du collagène du derme. En particulier, les expositions au soleil, les variations de température, la pollution, jouent un rôle néfaste sur la peau en générant des radicaux libres qui sont des dérivés réactifs de l'oxygène (ou ROS, de l'expression anglophone « *Reactive oxygen species* ») telles que l'anion superoxyde ($O_2^{\bullet-}$). L'attaque par voie radicalaire initie des réactions en chaîne qui ne s'arrêtent que lorsque deux radicaux libres s'inactivent mutuellement. En particulier, les radicaux libres génèrent un stress oxydant altérant les lipides membranaires des cellules de la peau et les cellules elles-mêmes, notamment au niveau de l'ADN mitochondrial et des mitochondries provoquant un vieillissement prématuré et/ou accéléré de la peau. En effet, la mitochondrie agit au sein de la cellule comme une centrale énergétique en produisant de l'ATP. La diminution de son activité, notamment liée à sa capacité à répondre au stress oxydatif sous la forme de ROS, est directement reliée au processus de vieillissement et aux symptômes de l'âge.

L'organisme aérobie possède heureusement un système de défense efficace contre le stress oxydant. En effet, de nombreux antioxydants naturels (enzymes et vitamines) aux propriétés anti-radicalaires sont soit produits par l'organisme, soit tirés de l'alimentation. Ainsi, la vitamine C inhibe les radicaux libres à l'intérieur de la cellule alors que la vitamine E et les caroténoïdes jouent le même rôle au niveau de la membrane cellulaire.

Le vieillissement cellulaire peut se définir comme la perte progressive de la capacité de l'organisme à maintenir l'équilibre entre radicaux libres et antioxydants. Au niveau de la peau, les radicaux libres induisent les marques du temps qui s'écoule : le flétrissement de la peau, le relâchement cutané, les rides, les taches pigmentaires... et également les cancers de la peau.

Il est donc important de protéger les cellules de la peau des effets néfastes des agressions de l'environnement, et en particulier du stress oxydatif généré notamment par les rayonnements UV et la pollution, afin de prévenir et/ou retarder l'apparition de signes de vieillissement cutanés tels que rides et ridules.

L'introduction d'antioxydants dans de nombreuses compositions cosmétiques à application topique telles que crèmes, laits, sérums, etc..., a pour objectif de combattre les effets néfastes de ces radicaux libres.

Il est donc possible de palier ce déséquilibre par l'application sur la peau de compositions cosmétiques ayant des propriétés anti-radicalaires afin de lutter contre l'apparition des signes du vieillissement.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle et présentant des propriétés anti-radicalaires.

De telles compositions contiennent classiquement au moins une substance antioxydante, en particulier des molécules complexes d'origine végétale telles que par exemple les polyphénols, les flavonoïdes, les anthocyanosides, les caroténoïdes, etc...

Cependant, malgré les diverses compositions disponibles, il existe toujours un besoin de pouvoir disposer de nouvelles compositions topiques alternatives permettant de lutter efficacement contre les effets du vieillissement cutané, et notamment des compositions topiques à base d'extraits végétaux appropriés provenant préférentiellement de plantes connues pour leurs propriétés favorables.

La passiflore, de la famille des *Passifloraceae*, est une plante médicinale vivace utilisée en infusions pour son effet calmant et sédatif. Elle comprend plusieurs centaines d'espèces, dont certaines, telles que *Passiflora edulis* et *Passiflora ligularis*, sont connues pour donner des fruits comestibles (fruit de la passion et grenadelle). Des compositions cosmétiques destinées à lutter contre le vieillissement de la peau et contenant des huiles riches en acides gras essentiels ainsi qu'un extrait de passiflore sont décrites dans le brevet EP 1 002 524. La demande de brevet JP 2002 332224 décrit une composition cosmétique pour lutter contre le vieillissement cutané contenant un extrait de passiflore à titre de composé permettant d'éliminer les ions superoxydes et un additif choisi parmi les agents hydratants, les antioxydants, les activateurs de cellules, les agents de blanchiment et les filtres solaires. Enfin, le brevet EP 2 291 173 décrit une composition cosmétique ou dermatologique comprenant en association un extrait de passiflore et un extrait d'*Anchusa arvensis*, procurant un effet décontractant de la peau et capable d'agir contre les contractions musculaires, en particulier pour prévenir ou réduire les rides d'expression résultant des contractions musculaires faciales incontrôlées.

L'edelweiss (en particulier *Leontopodium alpinum*), est une plante de la famille des Astéracées (anciennes Composées). C'est une des plus célèbres plantes de montagne, en partie en raison de sa rareté. L'edelweiss est cultivé en Suisse et est utilisé par l'industrie cosmétique pour ses propriétés anti-inflammatoires. Par ailleurs, le brevet FR 3031454 décrit l'utilisation de cellules indifférenciées ou dédifférenciées de *Leontopodium alpinum* obtenues par un procédé de culture cellulaire *in vitro* pour un traitement cosmétique pour restaurer l'homéostasie des cellules de la peau âgée et accroitre leur activité métabolique.

Or, les études effectuées par la Demanderesse ont montré que la combinaison d'un extrait de passiflore et de cellules de culture de callus d'edelweiss et/ou d'un extrait de cellules de culture de callus d'edelweiss, présente, de façon inattendue, une activité synergique antioxydante en présence d'un stress oxydatif rendant cette combinaison particulièrement utile à titre d'ingrédient dans une composition cosmétique destinée à la protection de la peau et à la prévention des signes du vieillissement cutané.

La présente invention a donc pour premier objet une composition cosmétique à application topique comprenant :

- au moins un extrait de passiflore, et
- au moins des cellules de culture de callus d'edelweiss et/ou au moins un extrait de cellules de culture de callus d'edelweiss, et
- au moins un support physiologiquement acceptable.

De préférence, les cellules de culture de callus d'edelweiss peuvent être des cellules entières. En d'autres termes, les cellules entières sont avantageusement des cellules non lysées.

Ainsi, ladite composition cosmétique peut comprendre au moins des cellules entières de culture de callus d'edelweiss et/ou au moins un extrait de cellules entières de culture de callus d'edelweiss.

On entend par « extrait de cellules entières de culture de callus d'edelweiss » un extrait dans lequel les cellules entières ont été notamment homogénéisées sous haute pression pour casser les agrégats cellulaires, et/ou lyophilisées.

Une telle composition cosmétique présente d'excellentes propriétés anti-radicalaires ces propriétés étant utilisables en cosmétique pour les soins de la peau, plus particulièrement pour protéger la peau des agressions de l'environnement, en particulier des effets néfastes des rayonnements UV ou de la pollution, et pour prévenir les signes du vieillissement cutané.

En particulier, les essais effectués par la Demanderesse ont montré que la combinaison d'un extrait de passiflore, en particulier de *Passiflora incarnata*, et d'un extrait de cellules de culture de callus d'edelweiss, en particulier de *Leontopodium alpinum*, permet de réduire le niveau d'oxydation intracellulaire en condition de stress oxydatif sur des fibroblastes humains et que cette activité se manifeste de façon synergique dans la gamme de concentrations testées.

L'invention a donc également pour second objet, un procédé cosmétique non thérapeutique pour le soin de la peau, notamment pour protéger la peau des agressions de l'environnement, prévenir, retarder et/ou combattre les signes du vieillissement cutané, consistant à appliquer sur les zones de la peau concernées au moins une composition cosmétique à application topique telle que définie ci-dessus dans le premier objet de l'invention, c'est-à-dire une composition cosmétique topique comprenant :

- au moins un extrait de passiflore, et
- au moins des cellules de culture de callus d'edelweiss et/ou au moins un extrait de cellules de culture de callus d'edelweiss, et

- au moins un support physiologiquement acceptable.

De préférence, les cellules de culture de callus d'edelweiss peuvent être des cellules entières. En d'autres termes, les cellules entières sont avantageusement des cellules non lysées.

Ainsi, ladite composition cosmétique peut comprendre au moins des cellules entières de culture de callus d'edelweiss et/ou au moins un extrait de cellules entières de culture de callus d'edelweiss.

On entend par « extrait de cellules entières de culture de callus d'edelweiss » un extrait dans lequel les cellules entières ont été notamment homogénéisées sous haute pression pour casser les agrégats cellulaires et/ou lyophilisées.

Enfin, l'invention a pour troisième objet l'utilisation non thérapeutique d'une composition cosmétique à application topique telle que définie ci-dessus selon le premier objet de l'invention, c'est-à-dire comprenant :

- au moins un extrait de passiflore, et
- au moins des cellules de culture de callus d'edelweiss et/ou au moins un extrait de cellules de culture de callus d'edelweiss, et
- au moins un support physiologiquement acceptable pour protéger la peau des agressions de l'environnement, prévenir, retarder et/ou combattre les signes du vieillissement cutané.

De préférence, les cellules de culture de callus d'edelweiss peuvent être des cellules entières. En d'autres termes, les cellules entières sont avantageusement des cellules non lysées.

Ainsi, ladite composition cosmétique peut comprendre au moins des cellules entières de culture de callus d'edelweiss et/ou au moins un extrait de cellules entières de culture de callus d'edelweiss.

On entend par « extrait de cellules entières de culture de callus d'edelweiss » un extrait dans lequel les cellules entières ont été notamment homogénéisées sous haute pression pour casser les agrégats cellulaires et/ou lyophilisées.

Au sens de la présente invention, on entend par « support physiologiquement acceptable », un support convenant pour une utilisation par voie topique ou par voie orale et dont la mise en contact avec les muqueuses, les ongles, le cuir chevelu, les cheveux, les poils et la peau de mammifère, et plus particulièrement des êtres humains, ne présente aucun risque de toxicité, d'incompatibilité, etc. Ce milieu physiologiquement acceptable forme ce que l'on appelle généralement l'excipient de la composition. Selon la présente invention, le support physiologiquement acceptable est de préférence un support cosmétiquement acceptable.

Par support cosmétiquement acceptable, on entend un support compatible avec la peau et/ou ses phanères, qui présente une couleur, une odeur et un toucher agréables et qui ne génère pas d'inconforts inacceptables (picotements, tiraillements, rougeurs), lors de son utilisation, notamment lors de son application sur la peau.

Parmi les diverses espèces du genre passiflore utilisables dans l'invention, on peut citer en particulier *Passiflora incarnata, Passiflora acuminata, Passiflora alata, Passiflora antioquensis, Passiflora antiquiensis, Passiflora aurantia, Passiflora baraquiniana, Passiflora biflora, Passiflora caerulea, Passiflora capsularis, Passiflora cincinatta, Passiflora coccinea, Passiflora cumbalensis, Passiflora dictamo, Passiflora edulis, Passiflora garckei, Passiflora glandulosa, Passiflora hastata, Passiflora hircina, Passiflora laurifolia, Passiflora ligularis, Passiflora mexicana, Passiflora mixta, Passiflora mollissima, Passiflora morifolia, Passiflora multiflora, Passiflora nitida, Passiflora ornithoura, Passiflora quadrangularis, Passiflora saponaria, Passiflora suberosa, Passiflora sulcata, Passiflora tenuiloba, Passiflora tinifolia, Passiflora vitifolia, Passiflora foetida, Passiflora nigelliflora, Passiflora obscura, Passiflora gossypifolia, Passiflora grandiflora, Passiflora hastada, Passiflora hibisciflora, Passiflora hirsina, Passiflora hirsuta, Passiflora hispida,* et leurs mélanges.

Suivant l'invention, on utilise de préférence l'espèce *Passiflora incarnata.*

On utilise de préférence les parties aériennes de passiflore, en particulier les sommités fleuries de passiflore, les sommités fleuries comprenant les fleurs et jeunes feuilles de la partie apicale, de préférence aux autres parties des plantes, pour préparer les extraits utilisables dans les compositions cosmétiques conforme à l'invention.

Les extraits de passiflore peuvent être préparés à partir de la plante séchée et broyée, la matière sèche ainsi obtenue représentant environ 5% du poids total de la plante. On peut par exemple préparer des extraits aqueux, des extraits alcooliques ou des extraits hydroalcooliques, par exemple des extraits hydro-glycoliques ou hydro-éthanoliques, selon les techniques bien connues de l'homme du métier, en introduisant de 0,5 à 3 % en masse, de préférence de 1 à 1,5 % en masse, de matière sèche de passiflore dans un solvant choisi parmi l'eau, un alcool et leurs mélanges. Ainsi, selon l'invention, la quantité de passiflore présente dans l'extrait utilisé est exprimée en masse de matière sèche de passiflore séchée et broyée introduite dans le solvant d'extraction choisi.

Les extraits de passiflore utilisables selon l'invention comprennent donc de préférence de 0,5 à 3 % en masse, et encore plus préférentiellement de 1 à 1,5 % en masse, de matière sèche de passiflore par rapport à la masse totale dudit extrait aqueux, alcoolique ou hydroalcoolique.

La quantité d'extrait de passiflore présente au sein de la composition cosmétique conforme à l'invention, exprimée en masse de matière sèche de passiflore introduite dans le solvant d'extraction, varie de préférence d'environ 0,001 à 0,5 %, plus préférentiellement d'environ 0,005 à 0,25 %, et encore plus préférentiellement de 0,01 à 0,05 % environ, par rapport à la masse totale de la composition cosmétique.

Parmi les diverses espèces d'edelweiss utilisables dans l'invention, on peut citer en particulier *Leontopodium alpinum, Leontopodium nivale, Leontopodium coreanum, Leontopodium discolor, Leontopodium haplophylloides, Leontopodium himalayanum, Leontopodium jacotianum, Leontopodium microphyllum, Gnaphalium leontopodium* et *Gnaphalium nivale,* et leurs mélanges.

Suivant une forme de réalisation préférée de l'invention, on utilise des cellules de culture de callus de *Leontopodium alpinum.*

Les cellules de callus d'edelweiss sont issues de la culture *in vitro* de cellules indifférenciées ou dédifférenciées, ci-après appelée culture cellulaire végétale.

Les méthodes *in vitro* de culture cellulaire sont bien connues de l'homme du métier. Elles comprennent généralement les étapes suivantes :

- l'établissement de lignées cellulaires à partir de callus (amas de cellules indifférenciées ou dédifférenciées) obtenus sur des coupures de parties de la plante (feuille, racine, tige, bourgeons, etc...),

- la sélection d'une lignée cellulaire capable de produire à grande échelle une biomasse de cellules selon des critères préétablis (phénotype constant et production optimale et constante de métabolites choisis, capacité à proliférer),

- la génération de ladite biomasse à partir de la lignée ainsi sélectionnée, éventuellement avec une étape d'élicitation, de préférence en fin de phase de prolifération,

- le traitement de la biomasse ainsi obtenue pour récupérer les cellules entières.

Selon la forme d'utilisation souhaitée, lesdites cellules peuvent ensuite être utilisées sous forme entière, éventuellement après homogénéisation sous haute pression pour casser les agrégats cellulaires, sous forme lyophilisée, ou bien subir une étape de lyse, pour en extraire le contenu cellulaire.

La quantité de cellules de culture de callus d'edelweiss au sein de la composition cosmétique conforme à l'invention varie généralement d'environ $7.10^{-4}$ à 0,3 %, de préférence de 0,0035 à 0,15 %, et encore plus préférentiellement d'environ 0,007 à 0,03%, ladite quantité étant exprimée en masse sèche par rapport à la masse totale de la composition cosmétique.

Selon l'invention, le rapport massique entre la quantité d'extrait de passiflore et la quantité de cellules de culture de callus d'edelweiss varie de préférence de 0,3 à 6 environ, et encore plus préférentiellement de 0,75 à 3.

Selon une forme de réalisation particulièrement préférée, la composition cosmétique comprend l'extrait de passiflore en une quantité d'environ 0,02125% en masse par rapport à la masse totale de ladite composition, ladite quantité étant exprimée en masse de matière sèche de passiflore introduite dans le solvant d'extraction, et 0,014% en masse de cellules de culture de callus d'edelweiss, par rapport à la masse totale de ladite composition.

La composition cosmétique selon l'invention peut contenir, outre l'extrait de passiflore et les cellules de culture de callus d'edelweiss, un ou plusieurs actifs secondaires renforçant ou complétant avantageusement son activité, et compatibles, c'est-à-dire non susceptibles de réagir les uns avec les autres ou de masquer ou limiter ses effets.

Plus particulièrement, les actifs secondaires peuvent être choisis par exemple parmi les agents hydratants, les agents humectants, les agents anti-oxydants, les agents de protection de la peau, les actifs anti-âge, les agents de protection contre les rayonnements, notamment UV et leurs mélanges.

Les agents hydratants peuvent par exemple être choisis parmi l'acide hyaluronique, les sels et copolymères de l'acide pyroglutamique, la lécithine hydrogénée, l'urée et ses dérivés, le cholestérol et leurs mélanges.

Les agents humectants peuvent par exemple être choisis parmi les glycols, la bétaïne, les sucres et dérivés de sucres, un extrait de ferment de *Pseudoalteromonas,* l'acide lactique, les acides aminés libres, les sels minéraux, les extraits végétaux comme par exemple la betterave, l'hibiscus, l'algue *Furcellaria lumbricalis* et leurs mélanges.

Les agents anti-oxydants peuvent par exemple être choisis parmi le tocophérol (Vitamine E) et ses dérivés ainsi que les extraits le contenant ; l'acide ascorbique (vitamine C) et ses dérivés ainsi que les extraits le contenant ; les phénols, polyphénols, acides phénoliques, flavonoïdes, lignanes, leurs dérivés ainsi que les extraits les contenant ; l'acide lipoïque et ses dérivés ainsi que les extraits le contenant ; les agents chélatants du fer et du cuivre ; la lactoferrine ; le coenzyme Q10 et leurs mélanges.

Les agents de protection de la peau peuvent être choisis par exemple parmi les agents favorisant la fonction barrière tels que les phospholipides, les céramides, les acides gras, le miel, des extraits végétaux tels que *Samanea saman,* jacinthe d'eau, rose de porcelaine, amarante, tara ; les agents anti-radicalaires ; les agents anti-pollution ; les agents anti-inflammatoires et apaisants tels que le panthénol, le résorcinol et dérivés, le bisabolol, l'allantoïne, le madécassoside, le menthol, les extraits de pivoine, de nymphéa, de camomille, de calendula, d'aloe et leurs mélanges.

Parmi les actifs anti-âge, on peut notamment citer les agents desquamants/exfoliants mécaniques ou chimiques ; les agents favorisant la microcirculation cutanée ; les agents dépigmentants tels que des extraits de cacao, de crocus et de tribulus ; les agents anti-glycation ; les agents raffermissants tels que les extraits de barbitamao ou de gleditsia ; les agents réparateurs tels que les extraits de camélia, de polygonum, de pommier ; les agents décontractants tels que les extraits de lotus bleu, de coquelicot, de guimauve, d'anchuse ; les agents stimulant la synthèse de macromolécules dermiques ou épidermiques et/ou empêchant leur dégradation en particulier les agents stimulant la prolifération des fibroblastes et/ou des kératinocytes tels que par exemple les peptides, lipopeptides et dérivés de peptides, des extraits végétaux tels que des extraits d'araucaria, de mimosa, de souci, des extraits d'algues, des extraits de plancton ; les rétinoïdes ; les agents tenseurs à base de protéines ou de polysaccharides végétaux et leurs mélanges.

Les agents de protection contre les rayonnements, notamment UV peuvent par exemple être choisis parmi les filtres organiques ainsi que parmi les filtres inorganiques et leurs mélanges.

Les compositions cosmétiques conformes à la présente invention peuvent se présenter sous les formes galéniques classiquement utilisées pour une application topique, par exemple sous forme de gel, d'émulsion (par exemple sous forme de crème ou de lait), de microémulsion, de sérum, d'huile, de masque, de pommade, d'onguent, de lotion, de solution concentrée, de suspension, de mousse, de bâtons solides, en particuliers de bâtons protecteurs pour les lèvres ou encore d'aérosols, lesdites formes galéniques contenant en outre des excipients et supports usuels et physiologiquement acceptables sur le plan cosmétologique.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile.

Au sein des compositions cosmétiques suivant l'invention, l'extrait de passiflore et/ou les cellules de culture de callus d'edelweiss et/ou un ou plusieurs des actifs secondaires peuvent également se présenter sous forme encapsulée dans des macro-, micro- ou nano-particules ou dans des macro-, micro- ou nanocapsules. L'extrait de passiflore et/ou les cellules de callus d'edelweiss et/ou un ou plusieurs des actifs secondaires peuvent être incorporés dans des vecteurs de type liposomes, glycosphères, dans des chylomicrons, des macro-, micro-, nano-particules ainsi que dans des macro-, micro-, nano-capsules et aussi être adsorbés sur un support choisi parmi les polymères organiques poudreux, les talcs, bentonites et autres supports minéraux.

Les compositions topiques selon l'invention peuvent comprendre des excipients appropriés pour une application topique externe, en particulier des excipients acceptables physiologiquement, et en particulier sur le plan cosmétologique. Ces excipients appropriés pour la formulation sont bien connus de l'homme du métier et comprennent par exemple les agents émollients, les solvants organiques, les agents chélatants, les agents de pénétration, les épaississants, les charges, les agents émulsionnants ou tensioactifs, les conservateurs, les colorants, les parfums et leurs mélanges.

Ainsi, ces compositions topiques peuvent être utilisées avantageusement en cosmétologie pour la prévention des signes du vieillissement cutané.

L'utilisation cosmétique de la composition cosmétique conforme à l'invention comprend tous les soins de la peau, des muqueuses, des phanères et du cuir chevelu, par exemple, les produits solaires, protecteurs et bronzants, les produits anti-âge, hydratants, anti-séborrhéiques, toniques, les produits assurant l'amélioration de l'aspect de la peau, notamment les produits blanchissants, les produits utilisés en parallèle à des traitements anti-acnéiques, les produits visant l'amélioration du confort cutané, par exemple les produits apaisants et/ou luttant contre les rougeurs et/ou la sécheresse cutanées.

Les exemples suivants illustrent l'invention plus en détail sans en limiter la portée. Dans tous les exemples de compositions qui suivent, les parties sont exprimées en masse, sauf indication contraire.

## EXEMPLES

L'extrait de passiflore utilisé dans les exemples illustrant la présente invention est un extrait hydro-glycériné obtenu à partir de l'espèce *Passiflora incarnata,* notamment à partir des sommités fleuries de cette espèce.

Après séchage et pulvérisation de la plante, l'extraction a été réalisée par percolation à raison de 100 g de plante pour 500 g d'eau. L'extrait aqueux obtenu par percolation est dilué dans 500 g de glycérine pour constituer un extrait hydro-glycériné utilisable en formulation cosmétique.

L'extrait ainsi obtenu présentait les caractéristiques suivantes :

| | |
|---|---|
| *Passiflora incarnata* (masse sèche) | 1,25% |
| Eau | 49,375% |
| Glycérine | 49,375% |

Il existe une équivalence directe entre la concentration exprimée en extrait hydro-glycériné tel que décrit ici, et la concentration exprimée en masse de matière sèche, avec 0,0425% en masse de matière sèche de Passiflore équivalant à 3,4% en masse de l'extrait.

L'extrait de cellules de culture de callus d'edelweiss utilisé dans les exemples illustrant l'invention est un extrait hydro-éthanolique obtenu à partir de l'espèce *Leontopodium alpinum.* Après culture cellulaire végétale, les cellules d'edelweiss sous forme lyophilisées ont été solubilisées dans un mélange de 70% d'éthanol et de 30% d'eau, puis la solution obtenue a été homogénéisée et filtrée.

L'extrait ainsi obtenu présentait les caractéristiques suivantes :

| | |
|---|---|
| *Cellules d'edelweiss* (masse sèche) | 21% |
| Ethanol | 55,3% |
| Eau | 23,7% |

Un extrait de cellules de culture de *Leontopodium alpinum* est par ailleurs commercialisé sous la dénomination commerciale Majestem® par la société Sederma. Plus particulièrement, cet extrait est un extrait de cellules entières de culture de callus d'edelweiss. Ce produit comprend outre les cellules de culture de callus de *Leontopodium alpinum,* de la glycérine et de la gomme de xanthane. Il existe une équivalence directe entre la concentration exprimée en masse de matière sèche et la concentration exprimée pour l'ingrédient commercial Majestem®, avec 0,028% en masse de cellules de callus d'Edelweiss équivalant à 2% en masse de Majestem®.

**EXEMPLE 1 : Mise en évidence de l'effet antioxydant de l'association d'un extrait de passiflore et de cellules de callus d'edelweiss**

Dans cet exemple, on a étudié les propriétés antioxydantes de l'association d'un extrait de passiflore et de cellules de callus d'edelweiss à différentes concentrations.

1.1. Principe du test

Le principe du test est basé sur la mesure du degré d'oxydation intracellulaire à l'aide d'une sonde spécifique : le diacétate de 2',7'-dichlorodihydrofluorescéine (DCFH-DA) qui est un indicateur sensible aux espèces réactives de l'oxygène (ou en anglais « *Reactive oxygen species* » : ROS). Le DCFH-DA pénètre dans le milieu intracellulaire où, après clivage des deux groupements acétates par les estérases intracellulaires, le DCFH produit s'accumule au niveau du cytosol.

L'oxydation intracellulaire du DCFH par différentes espèces réactives de l'oxygène (ROS) conduit à la formation d'un produit fluorescent, la 2',7'-dichlorofluorescéine (DCF). Plus l'échantillon testé est antioxydant, plus il préviendra l'oxydation du DCFH en DCF et moins le signal de fluorescence sera élevé. À l'inverse, si l'échantillon testé n'est pas antioxydant, la fluorescence se développera en réponse à un stress oxydatif induit. Le Contrôle Négatif, non traité, permet de connaître le taux d'oxydation basal des cellules. Le Contrôle Positif, traité par ajout de 5 $\mu$M d'un composé pro-oxydant, l'hydroperoxyde de *tert*-butyle (tBHP), permet de modéliser le stress oxydatif induit en l'absence d'antioxydant.

La mesure des intensités de fluorescence pour chaque lot testé permet alors d'évaluer le degré d'oxydation intracellulaire des cellules cutanées soumises à un stress oxydatif par ajout de 5 $\mu$M de tBHP en présence des extraits et donc l'activité antioxydante des extraits testés.

1.2. Protocole

L'étude a été réalisée sur des fibroblastes humains normaux issus d'une femme âgée de 46 ans (ATCC, CRL2106™). Les fibroblastes ont été cultivés dans du milieu de culture glucosé (milieu DMEM à 5 mM de glucose contenant 10 mM de tampon HEPES et 10% de sérum de veau foetal), placés à l'incubateur à 37°C et sous atmosphère air/$CO_2$ (95%/5%).

24 heures avant le début du test, les fibroblastes ont été transférés dans des plaques de 96 puits à raison de 20000 cellules par puits et mis en contact avec les traitements à l'étude puis replacés dans les mêmes conditions de culture.

Les traitements à l'étude ont été répartis en 13 lots décrits ci-dessous, dans lesquels les concentrations sont exprimées en masse de matière sèche de passiflore (PF) et d'edelweiss (EW). A partir des extraits décrits ci-dessus, la concentration a été ajustée par dilution dans le milieu de culture cellulaire.

Lot 1 : **TEMOIN (-)** : fibroblastes non traités et non stressés ;

**Lot 2** : **TEMOIN (+)** : fibroblastes non traités et stressés ;

**Lot 3** : **Extrait seul** : fibroblastes traités avec 0,010625% de matière sèche de passiflore (PF) et stressés ;

**Lot 4** : **Extrait seul** : fibroblastes traités avec 0,02125% de matière sèche de passiflore (PF) et stressés ;

**Lot 5** : **Extrait seul** : fibroblastes traités avec 0,0425% de matière sèche de passiflore (PF) et stressés ;

**Lot 6** : **Extrait seul** : fibroblastes traités avec 0,007% de cellules de culture de callus d'edelweiss (EW) et stressés ;

**Lot 7** : **Extrait seul** : fibroblastes traités avec 0,014% de cellules de culture de callus d'edelweiss (EW) et stressés ;

**Lot 8** : **Extrait seul** : fibroblastes traités avec 0,028% de cellules de culture de callus d'edelweiss (EW) et stressés ;

**Lot 9** : **Extraits combinés** : fibroblastes traités avec 0,010625% de matière sèche de passiflore (PF) et 0,014% de cellules de culture de callus d'edelweiss (EW) et stressés ;

**Lot 10** : **Extraits combinés** : fibroblastes traités avec 0,02125% de matière sèche de passiflore (PF) et 0,014% de cellules de culture de callus d'edelweiss (EW) et stressés ;

**Lot 11** : **Extraits combinés** : fibroblastes traités avec 0,0425% de matière sèche de passiflore (PF) et 0,014% de cellules de culture de callus d'edelweiss (EW) et stressés ;

**Lot 12** : **Extraits combinés** : fibroblastes traités avec 0,02125% de matière sèche de passiflore (PF) et 0,007% de cellules de culture de callus d'edelweiss (EW) et stressés ;

**Lot 13** : **Extraits combinés** : fibroblastes traités avec 0,02125% de matière sèche de passiflore (PF) et 0,028% de cellules de culture de callus d'edelweiss (EW) et stressés ;

Comme indiqué ci-dessus, les fibroblastes du Lot 1 n'ont reçu aucun traitement ni aucun stress (Contrôle négatif : non traité non stressé), alors que les fibroblastes du Lot 2 ont subi un stress oxydatif par ajout de 5 $\mu$M de tBHP (Contrôle positif : stress seul).

Pour chacun des lots 3 à 13, les fibroblastes ont été exposés simultanément aux quantités indiquées de chacun des extraits ainsi qu'au traitement par ajout de 5 $\mu$M de tBHP permettant d'induire le stress oxydatif.

A l'issue des 24 heures de traitement, la sonde DCFH-DA a été ajoutée dans chacun des puits, puis les plaques ont à nouveau été incubées à 37°C pendant 30 min. 10 $\mu$l de peroxyde d'hydrogène ($H_2O_2$) ont par ailleurs été ajoutés dans certains puits afin de vérifier la réponse et l'absence de saturation de la sonde. Les cellules ont ensuite été lavées deux fois à l'aide d'une solution tampon de PBS (*phosphate buffered saline*), puis la fluorescence a été mesurée à l'aide d'un spectrofluorimètre (CLARIOstar, BMG LABTEK). Dans un second temps, une normalisation de la densité optique a été réalisée par marquage des cellules avec une solution de cristal violet.

1.3. Analyse des données

Pour tous les lots (Lots 1 à 13), on calcule le pourcentage d'activité mesurée du lot par rapport au niveau basal des cellules représenté par le Contrôle Négatif (Lot 1) selon la formule (1) suivante :

$$\% \text{ ACTm Lot N} = 100 * \text{Valeur ROS Lot N} / \text{Valeur ROS CtrlNég} \qquad (1)$$

dans laquelle :

- % ACTm Lot N est le pourcentage d'activité mesurée pour chaque lot
- Valeur ROS Lot N est la valeur des ROS mesurée pour chaque lot
- Valeur ROS CtrlNég est la valeur des ROS mesurée pour le Contrôle Négatif (Lot 1).

Pour chaque combinaison (Lots 9 à 13), on évalue ensuite l'activité théorique équivalant au cumul des effets des deux composés en présence de stress. Pour cela, on calcule d'abord la contribution de chacun des deux composés seuls aux trois concentrations testées en présence de stress (Lots 3 à 8) par rapport au niveau observé pour le stress seul (Contrôle Positif - Lot 2) selon la formule (2) suivante pour l'extrait de passiflore (PF) :

$$\text{Contrib PF [conc x]} = \text{Valeur ROS PF [conc x]} / \text{Valeur ROS CtrlPos} \qquad (2)$$

dans laquelle :

- Contrib PF [conc x] est la contribution de l'extrait de passiflore à la concentration x,
- Valeur ROS PF [conc x] est la valeur des ROS mesurée pour le lot traité par l'extrait de passiflore à la concentration x,
- Valeur ROS CtrlPos est la valeur des ROS mesurée pour le Contrôle Positif (Lot 2) ;

et selon la formule (3) suivante pour les cellules de culture de callus d'edelweiss (EW) :

$$\text{Contrib EW [conc y]} = \text{Valeur ROS EW [conc y]} / \text{Valeur ROS CtrlPos} \qquad (3)$$

dans laquelle :

- Contrib EW [conc y] est la contribution des cellules de culture de callus d'edelweiss à la concentration y,
- Valeur ROS EW [conc y] est la valeur des ROS mesurée pour le lot traité par les cellules de culture de callus d'edelweiss à la concentration y,
- Valeur ROS CtrlPos est la valeur des ROS mesurée pour le Contrôle Positif (Lot 2).

Pour chaque combinaison testée, l'activité théorique devrait être équivalente au cumul des contributions de chaque composé seul à la concentration utilisée dans la combinaison, par rapport à l'activité du stress seul (Contrôle Positif - Lot 2). Le pourcentage d'activité théorique pour les combinaisons est donc calculé selon la formule (4) suivante :

$$\text{\% ACTth Combi PF [conc x] EW [conc y]} = \text{\% ACTm CtrlPos} * \text{Contrib PF [conc x]} * \text{Contrib EW [conc y]} \qquad (4)$$

dans laquelle :

- % ACTth Combi PF [conc x] EW [conc y] est le pourcentage d'activité théorique de la combinaison de l'extrait de passiflore testé à la concentration x et des cellules de culture de callus d'edelweiss à la concentration y,
- % ACTm CtrlPos est le pourcentage d'activité mesurée du Contrôle Positif,
- Contrib PF [conc x] et Contrib EW [conc y] sont tels que définis ci-dessus pour la formule (2) et la formule (3).

Les activités théoriques calculées ont été comparées aux activités mesurées pour chacune des combinaisons testées en présence de stress, aux valeurs des extraits seuls et aux valeurs des conditions Contrôle Négatif et Contrôle Positif.

4.4. Résultats

Les résultats obtenus sont reportés dans les tableaux I et II suivants :

**TABLEAU I**

| Lots | Contrôle négatif (Lot 1) | Contrôle Positif (Lot 2) | Extrait PF seul+ stress | Extrait EW seul + stress |
|---|---|---|---|---|
| 3 | 100 % | 225 % | 298 % | - |
| 4 | 100 % | 225 % | 190 % | - |
| 5 | 100 % | 225 % | 96 % | - |
| 6 | 100 % | 225 % | - | 216 % |
| 7 | 100 % | 225 % | - | 234 % |
| 8 | 100 % | 225 % | - | 197 % |

**TABLEAU II**

| LOTS | 9 | 10 | 11 | 12 | 13 |
|---|---|---|---|---|---|
| **Contrôle négatif (Lot 1)** | 100 % | 100 % | 100 % | 100 % | 100 % |
| **Contrôle Positif (Lot 2)** | 225 % | 225 % | 225 % | 225 % | 225 % |
| **Extrait EW+ stress** | 234 % | 234 % | 234 % | 216 % | 197 % |
| **Extrait PF + stress** | 298 % | 190 % | 96 % | 190 % | 190 % |
| **% d'activité Combinaison théorique** | 310 % | 198 % | 100 % | 182 % | 166 % |
| **% d'activité Combinaison mesurée** | 182 % | 68 % | 74 % | 165 % | 86 % |
| **% de Synergie** | **-128** % | **-130** % | **-26** % | **-17** % | **-80** % |

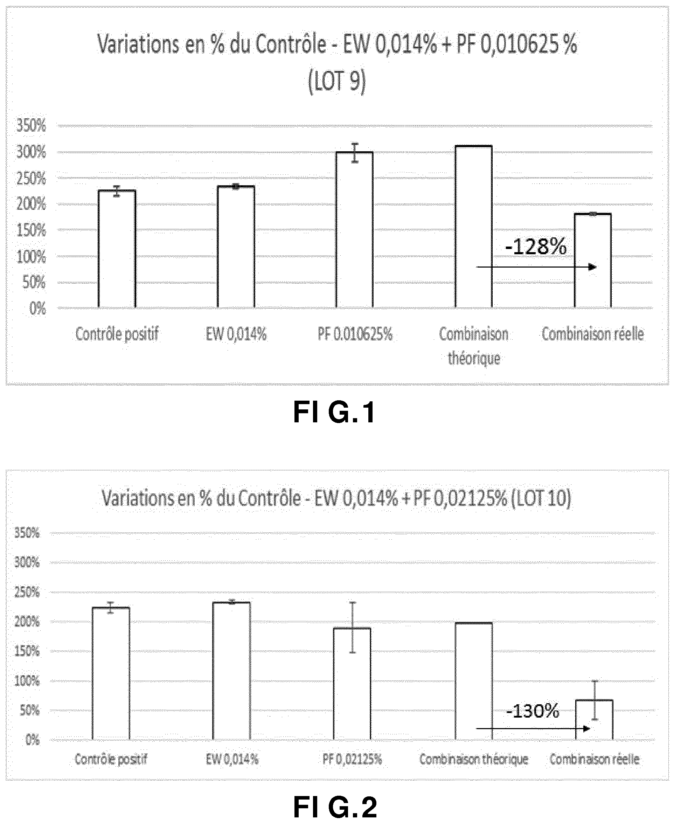

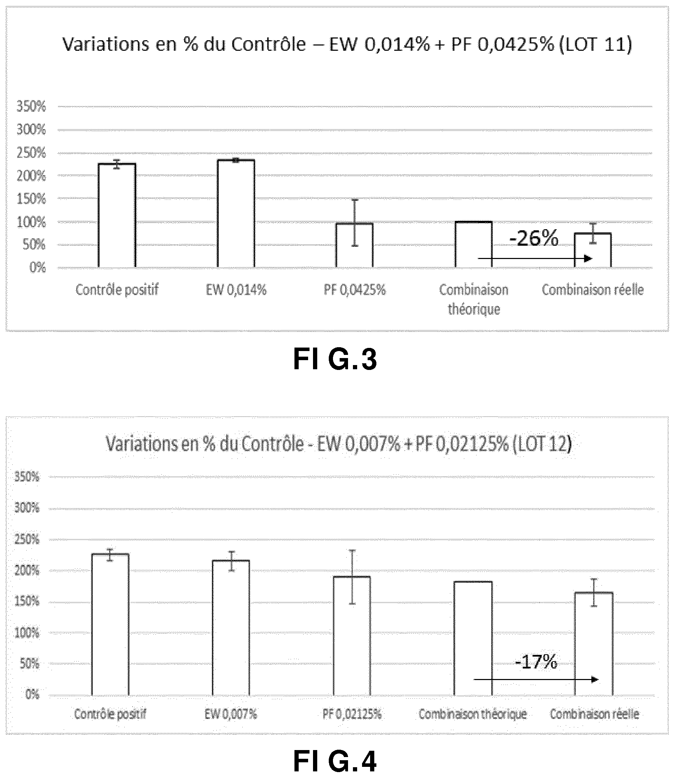

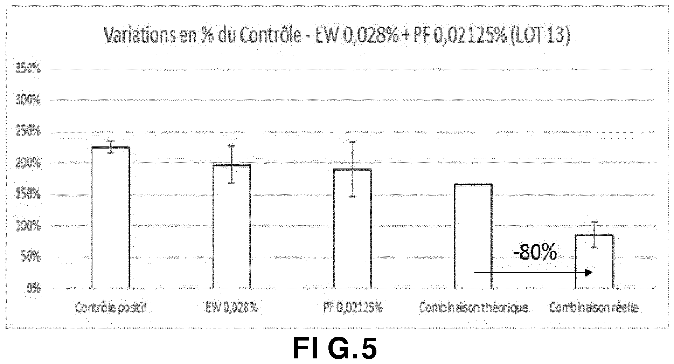

Les résultats obtenus avec les combinaisons des lots 9 à 13 sont également représentés sur les figures 1 à 5 annexées.

La condition Contrôle Positif (Lot 2) montre une augmentation de 125% de la quantité de ROS produits par rapport au Contrôle Négatif (Lot 1) mettant en évidence l'effet pro-oxydant du tBHP. Cette augmentation des ROS n'est pas rectifiée de manière significative par les composés seuls à l'exception de la composition contenant l'extrait de passiflore à 3,4%.

On peut remarquer que l'effet antioxydant mesuré de toutes les combinaisons testées est supérieur à l'effet théorique calculé de l'addition des extraits démontrant une action synergique des deux extraits lorsqu'ils sont utilisés en combinaison (Figures 1 à 5). Cette synergie est observée pour l'ensemble des combinaisons étudiées, pour des ratios de concentrations des deux extraits variables, tel que résumé dans le tableau II ci-dessus.

La synergie la plus forte est observée pour l'association PF à 0,02125% et EW à 0,014% (Lot 10), qui montre une réduction par un facteur 3 du niveau de ROS mesuré en comparaison à la condition stress seul (Contrôle Positif), ainsi qu'un niveau de ROS inférieur au niveau basal des cellules (Contrôle Négatif), mettant en évidence un effet antioxydant très fort de cette combinaison.

## EXEMPLE 2 : LOTION ANTI-AGE

Par les techniques usuelles, on a préparé une lotion anti-âge conforme à la présente invention et comprenant les ingrédients suivants (en % massique) :

| | |
|---|---|
| - Gomme de xanthane | 0,1 à 0,2 % |
| - Gluconate de sodium | 0,1 à 0,5 % |
| - Extrait hydro-glycériné de passiflore à 1,25% en masse de matière sèche de Passiflore | 0,85 à 3,40 % |
| - Extrait de cellules de culture de *Leontopodium alpinum* commercialisé sous la dénomination commerciale Majestem® par la société Sederma | 0,5 à 2 % |
| - Glycérine | 1 à 10 % |
| - Alcool dénaturé | 1 à 5 % |
| - L-arginine | qs |
| - Acide citrique, monohydrate | qs |
| - Conservateurs | qs |
| - Solubilisants | qs |
| - Parfum | qs |
| - Eau déminéralisée    qsp | 100,00% |

## EXEMPLE 3 : CREME ANTI-AGE

Par les techniques usuelles, on a préparé une crème anti-âge conforme à la présente invention et comprenant les ingrédients suivants (en % massique) :

| | |
|---|---|
| - Cetyl alcohol, Glyceryl stearate, PEG-75 stearate, Ceteth-20, Steareth-20 | 1 à 7 % |
| - Caprylic/Capric Triglyceride | 1 à 15 % |

(suite)

| | |
|---|---|
| - Hexyldecyl laurate, Hexyldecanol | 1 à 15 % |
| - Carbomer | 0,2 à 1 % |
| - Gluconate de sodium | 0,1 à 0,5 % |
| - Polyacrylate de sodium | 0,05 à 0,2 % |
| - Tocophérol | 0,05 à 1 % |
| - Alcool dénaturé | 1 à 5 % |
| - Extrait hydro-glycériné de passiflore à 1,25% en masse de matière sèche de Passiflore | 0,85 à 3,40 % |
| - Extrait de cellules de culture de *Leontopodium alpinum* commercialisé sous la dénomination commerciale Majestem® par la société Sederma | 0,5 à 2,00 % |
| - Hydroxyde de sodium | qs |
| - Acide citrique, monohydrate | qs |
| - Conservateurs | qs |
| - Parfum | qs |
| - Eau déminéralisée | qsp 100,00% |

## Revendications

1. Composition cosmétique à application topique comprenant :

   - au moins un extrait de passiflore, et
   - au moins des cellules de culture de callus d'edelweiss et/ou au moins un extrait de cellules de culture de callus d'edelweiss, et
   - au moins un support physiologiquement acceptable.

2. Composition cosmétique selon la revendication 1, **caractérisée en ce que** l'extrait de passiflore est préparé à partir des parties aériennes de passiflore, et de préférence à partir des sommités fleuries de passiflore.

3. Composition cosmétique selon la revendication 1 ou 2, **caractérisée en ce que** l'extrait de passiflore est un extrait aqueux, un extrait alcoolique ou un extrait hydroalcoolique.

4. Composition selon la revendication 3, **caractérisée en ce que** ledit extrait de passiflore est obtenu par introduction de 0,5 à 3 % en masse de matière sèche de passiflore séchée et broyée dans un solvant d'extraction choisi parmi l'eau, un alcool et leurs mélanges.

5. Composition selon la revendication 4, **caractérisée en ce que** la quantité d'extrait de passiflore, exprimé en masse de matière sèche de passiflore introduite dans ledit solvant d'extraction, varie de 0,001 à 0,5 % en masse, par rapport à la masse totale de ladite composition.

6. Composition selon la revendication 4 ou 5, **caractérisée en ce que** la quantité d'extrait de passiflore, exprimée en masse de matière sèche de passiflore introduite dans ledit solvant d'extraction, varie de 0,005 à 0,25 % en masse, par rapport à la masse totale de ladite composition.

7. Composition selon l'une quelconque des revendications 1 à 6, **caractérisée en ce qu'**elle comprend des cellules de culture de callus d'edelweiss en une quantité de $7.10^{-4}$ à 0,3 %, ladite quantité étant exprimée en masse de matière sèche par rapport à la masse totale de ladite composition.

8. Composition selon l'une quelconque des revendications 1 à 7, **caractérisée en ce qu'**elle comprend des cellules de culture de callus d'edelweiss en une quantité de 0,0035 à 0,15 % ladite quantité étant exprimée en masse sèche par rapport à la masse totale de ladite composition.

9. Composition selon l'une quelconque des revendications 1 à 8, **caractérisée en ce que** le rapport massique entre la quantité d'extrait de passiflore et la quantité de cellules de culture de callus d'edelweiss varie de 0,3 à 6.

10. Composition selon l'une quelconque des revendications 4 à 9, **caractérisée en ce que** ladite composition comprend l'extrait de passiflore en une quantité de 0,02125% en masse par rapport à la masse totale de ladite composition, ladite quantité étant exprimée en masse de matière sèche de passiflore introduite dans ledit solvant d'extraction, et les cellules de culture de callus d'edelweiss en une quantité de 0,014%, ladite quantité étant exprimée en masse sèche par rapport à la masse totale de ladite composition.

11. Composition selon l'une quelconque des revendications 1 à 10, **caractérisée en ce qu'**elle contient en outre un ou plusieurs actifs secondaires choisis parmi les agents hydratants, les agents humectants, les agents anti-oxydants, les agents de protection de la peau, les actifs anti-âge, les agents de protection contre les rayonnements, notamment UV et leurs mélanges.

12. Composition selon l'une quelconque des revendications 1 à 11, **caractérisée en ce que** l'extrait de passiflore et/ou les cellules de culture de callus d'edelweiss et/ou un ou plusieurs des actifs secondaires se présente(nt) sous forme encapsulée dans des macro-, micro-, ou nano-particules ou dans des macro-, micro-, ou nanocapsules, ou adsorbés sur un support choisi parmi les polymères organiques poudreux, les talcs, les bentonites et autres supports minéraux.

13. Composition selon l'une quelconque des revendications 1 à 12, **caractérisée en ce qu'**elle se présente sous la forme de gel, d'émulsion, de microémulsion, de sérum, d'huile, de masque, de pommade, d'onguent, de lotion, de solution concentrée, de suspension, de mousse, de bâtons solides ou d'aérosols.

14. Procédé cosmétique non thérapeutique pour le soin de la peau, notamment pour protéger la peau des agressions de l'environnement, prévenir, retarder et/ou combattre les signes du vieillissement cutané, consistant à appliquer sur les zones de la peau concernées au moins une composition cosmétique à application topique telle que définie à l'une quelconque des revendications 1 à 13.

15. Utilisation non thérapeutique d'une composition cosmétique à application topique telle que définie à l'une quelconque des revendications 1 à 13, pour protéger la peau des agressions de l'environnement, prévenir, retarder et/ou combattre les signes du vieillissement cutané.

**FI G.1**

**FI G.2**

**Variations en % du Contrôle – EW 0,014% + PF 0,0425% (LOT 11)**

**FI G.3**

**Variations en % du Contrôle - EW 0,007% + PF 0,02125% (LOT 12)**

**FI G.4**

Variations en % du Contrôle - EW 0,028% + PF 0,02125% (LOT 13)

FIG.5

Europäisches
Patentamt

European
Patent Office

Office européen
des brevets

## RAPPORT DE RECHERCHE EUROPEENNE

Numéro de la demande

EP 19 19 0801

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 3 045 380 A1 (EXPANSCIENCE LAB [FR]) 23 juin 2017 (2017-06-23) * revendications 1,5 * * page 23, ligne 10 - page 24, ligne 8 * ----- | 1-15 | INV. A61Q19/08 A61K8/9794 |
| X | EP 2 319 914 A1 (I R B ISTITUTO DI RICERCHE BIOTECNOLOGICHE S R L [IT]) 11 mai 2011 (2011-05-11) * revendications 18,9,11 * * alinéa [0007] * * exemple 2 * ----- | 1-15 | |
| Y | DATABASE GNPD [Online] MINTEL; 2 mars 2016 (2016-03-02), anonymous: "Alp-Cells Repair Unique Global Anti-Aging Cream", XP055536024, extrait de www.gnpd.com Database accession no. 3735105 * abrégé * ----- | 1-15 | |
| X | DATABASE GNPD [Online] MINTEL; 23 mai 2016 (2016-05-23), anonymous: "Day Cream for Dry Skin", XP055536032, extrait de www.gnpd.com Database accession no. 3999883 * abrégé * ----- -/-- | 1-15 | DOMAINES TECHNIQUES RECHERCHES (IPC) A61Q A61K |

Le présent rapport a été établi pour toutes les revendications

| Lieu de la recherche | Date d'achèvement de la recherche | Examinateur |
|---|---|---|
| Munich | 16 septembre 2019 | Verrucci, Marinella |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

page 1 de 2

Europäisches
Patentamt

European
Patent Office

Office européen
des brevets

**RAPPORT DE RECHERCHE EUROPEENNE**

Numéro de la demande

EP 19 19 0801

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DATABASE GNPD [Online] MINTEL; 5 juin 2017 (2017-06-05), anonymous: "Facial Cream", XP055536036, extrait de www.gnpd.com Database accession no. 4863989 * abrégé * ----- | 1-15 | |
| A | DATABASE GNPD [Online] MINTEL; 7 avril 2017 (2017-04-07), anonymous: "Antioxidant Moisturizer with White Tea", XP055536041, extrait de www.gnpd.com Database accession no. 4678057 * abrégé * ----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 16 septembre 2019 | Verrucci, Marinella |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
..................................................................
& : membre de la même famille, document correspondant

EPO FORM 1503 03.82 (P04C02)

page 2 de 2

**ANNEXE AU RAPPORT DE RECHERCHE EUROPEENNE
RELATIF A LA DEMANDE DE BREVET EUROPEEN NO.**

EP 19 19 0801

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-2019

| Document brevet cité au rapport de recherche | | Date de publication | Membre(s) de la famille de brevet(s) | | Date de publication |
|---|---|---|---|---|---|
| FR 3045380 | A1 | 23-06-2017 | CN | 108697610 A | 23-10-2018 |
| | | | EP | 3393444 A1 | 31-10-2018 |
| | | | FR | 3045380 A1 | 23-06-2017 |
| | | | JP | 2018538332 A | 27-12-2018 |
| | | | KR | 20180121877 A | 09-11-2018 |
| | | | US | 2019000902 A1 | 03-01-2019 |
| | | | WO | 2017108978 A1 | 29-06-2017 |
| EP 2319914 | A1 | 11-05-2011 | AUCUN | | |

EPO FORM P0460

Pour tout renseignement concernant cette annexe : voir Journal Officiel de l'Office européen des brevets, No.12/82

**EP 3 607 997 A1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1002524 A
- JP 2002332224 A
- EP 2291173 A
- FR 3031454